**Europäisches Patentamt**

**European Patent Office**

**Office européen des brevets**

(19)

(11) Veröffentlichungsnummer: **0 011 263**
**A1**

# (12) EUROPÄISCHE PATENTANMELDUNG

(21) Anmeldenummer: **79104434.0**

(22) Anmeldetag: **12.11.79**

(51) Int. Cl.³: **C 09 K 3/30**
**A 61 K 9/12**

(30) Priorität: **11.11.78 DE 2849074**

(43) Veröffentlichungstag der Anmeldung:
**28.05.80 Patentblatt 80/11**

(84) Benannte Vertragsstaaten:
**BE FR GB IT NL SE**

(71) Anmelder: **Wittenhorst, Augustinus J.M.**
**Langenhahner Strasse 33**
**D-5438 Westerburg(DE)**

(71) Anmelder: **VON TREU AG**
**Chamer Strasse 79**
**CH-6300 Zug 3(CH)**

(72) Erfinder: **Wittenhorst, Augustinus J.M.**
**Langenhahner Strasse 33**
**D-5438 Westerburg(DE)**

(74) Vertreter: **Grommes, Karl F., Dr.**
**Casinostrasse 37**
**D-5400 Koblenz(DE)**

(54) **Aerosolerzeugnis, Verfahren zur Herstellung eines Aerosolerzeugnisses und Vorrichtung zur Durchführung des Verfahrens.**

(57) Das *Aerosolerzeugnis*, beispielsweise ein pharmazeutisches oder kosmetisches Präparat, enthält Wirkstoff, Treibmittel und Lösungsmittel, wobei das Treibmittel zur Abwehr von Schäden an der Umwelt sowie bei den Benutzern ausschließlich aus Helium, Neon, Argon, Krypton oder Gemischen davon besteht. Als besonders günstig gilt ein Anteil an Argon von mindestens 80 Gew.%, bezogen auf das Gesamtgewicht des Treibmittels.

Das *Verfahren* sieht im wesentlichen vor, daß Wirkstoff und Lösungsmittel in einem Druckbehälter verrieselt werden, wobei ein komprimiertes Edelgas zudosiert und anschließend das Bemisch gesammelt wird. Der Mischvorgang kann in weiteren Druckbehältern mit verschiedenen Edelgasen wiederholt werden.

Die *Vorrichtung* umfaßt Mischkessel (1, 2; 3) zur Speicherung von Wirkstoff und Lösungsmittel, wobei diese ebenso wie ein Druckspreicher (12a) für Edelgas an einen Druckbehälter (12) angeschlossen sind, bei welchem ein Abgangsstutzen (14) im Abstand vom Boden angeordnet ist. Es können mehrere Druckbehälter (15, 18) vorgesehen sein, ebenso ein Kerzenfilter (22, 10a) hinter dem letzten Druckbehälter (18) und/oder vor dem ersten Druckbehälter (12).

EP 0 011 263 A1

=================================================

Aerosolerzeugnis, Verfahren zur Herstellung eines
Aerosolerzeugnisses und Vorrichtung zur Durchführung des Verfahrens

=================================================

## Technisches Gebiet

Die Erfindung betrifft ein Aerosolerzeugnis, bestehend aus Wirkstoff, Treibmittel und Lösungsmittel. Ferner betrifft die Erfindung ein Verfahren
zur Herstellung eines Aerosolerzeugnisses sowie
eine Vorrichtung zur Durchführung des Verfahrens.

Stand der Technik

Es sind Aerosolerzeugnisse mit verschiedenen Treibmitteln bekannt. Da die Treibmittel sich beim Versprühen verflüchtigen und dabei leicht bis hin zu offenen Brennstellen gelangen können, wird von ihnen im allgemeinen gefordert, daß ihre Brennbarkeit herabgemindert ist.

Ein bekanntes Beispiel für derartige Treibmittel sind Fluor-Chlor-Kohlenwasserstoffe. Ihr Gebrauch erscheint allerdings nicht ganz unbedenklich, da zumindest die Möglichkeit besteht, daß bei einem anhaltenden und größeren Ausstoß in die Erdatmosphäre ein Angriff auf den die Erde umgebenden Ozongürtel erfolgt, welcher für die Absorption von UV-Strahlen Bedeutung besitzt.

Es sind auch fluorkohlenwasserstofffreie Treibmittel bekannt (vgl. DE-OS 2 741 882), nämlich mit 65 bis 85 Gew.% 1,1,1-Trichloräthan, Dichlormethan oder Gemischen davon, 14 bis 32 Gew.% Butan, Isobutan, Propan oder Gemischen davon und etwa 0,5 bis 5,3 Gew.% Kohlendioxid. Nachteilig daran ist für viele Anwendungsbereiche der hohe Anteil an 1,1,1-Trichloräthan bzw. Dichlormethan. Zum einen unterliegen jene Komponenten beim Sprühen in eine offene Flamme, wie es regelmäßig bei Laborversuchen (Flammtests u.ä.), darüber hinaus aber auch unbeabsichtigt beim praktischen Gebrauch vorkommt, Zersetzungsreaktionen, wobei unter anderem Salzsäure entstehen kann, zum anderen ist es auch ohne, daß eine Zersetzung stattfindet, bedenklich, wenn ein

Benutzer in direkte Berührung mit jenen Komponenten gerät, insbesondere diese mit inhaliert. Das kommt vor allem für Aerosolerzeugnisse auf dem Gebiet der Pharmazie, aber auch für zahlreiche andere Aerosolerzeugnisse in Betracht, da deren Freisetzung praktisch immer in unmittelbarer Nähe des Benutzers erfolgt.

Darstellung der Erfindung

Hier setzt nun die Erfindung an. Ihr liegt die Aufgabe zugrunde, ein Aerosolerzeugnis anzugeben, von dem diese Gefahren nicht mehr ausgehen. Das Aerosolerzeugnis soll sich also, soweit es sein Treibmittel anbelangt, sowohl durch verminderte Brennbarkeit als auch neutrales Verhalten bezüglich der Umwelt wie auch der unmittelbaren Gesundheit des Benutzers auszeichnen. Ferner soll die Erfindung ein Verfahren zur Herstellung eines solchen Aerosolerzeugnisses angeben wie auch eine Vorrichtung zur Durchführung des Verfahrens.

Gelöst wird die Aufgabe bei einem aus Wirkstoff, Treibmittel und Lösungsmittel bestehenden Aerosolerzeugnis auf überraschend einfache und gleichzeitig wirksame Weise dadurch, daß das Treibmittel ausschließlich aus Helium, Neon, Argon, Krypton oder Gemischen davon besteht.

Das gilt sowohl für Pudersprays, Schaumsprays als auch vor allem für Netzsprays. Die Auswahl der genannten Edelgase richtet sich zum einen nach deren Preiswürdigkeit, zum anderen nach ihren physikalischen Eigenschaften.

Aus der DE-AS 1 170 909 sind selbsttreibende Gemische bekannt, die als Treibmittel ein Inertgas und ein halogeniertes Methan enthalten. Dabei besteht das Inertgas aus einem Gemisch aus Schwefelhexafluorid und einem inerten, nicht brennbaren Gas, z.B. auch Argon, Helium oder Neon.

Die DE-OS 21 31 668 betrifft anaerobe Mittel, insbesondere in Abwesenheit von Sauerstoff abbindende Kleber, in Aerosolform. Neben dem "anaeroben Mittel" sind in den unter Druck stehenden Behältern ein Inertgas und Sauerstoff vorhanden. Neben seiner chemischen Funktion wirkt der Sauerstoff dabei auch als Treibmittel. Als Inertgase sind unter anderem auch Helium, Neon und Argon erwähnt.

Aus keiner der vorgenannten Druckschriften geht die Verwendung von Edelgasen als alleinigem Treibmittel für Aerosole hervor.

Edelgase sind absolut unbrennbar und damit den bisherigen Treibmitteln überlegen. Ferner bringen sie keine Gefahr für die Erdatmosphäre mit sich, sondern sind insgesamt als umweltfreundlich einzustufen. Bekanntermaßen werden sie nämlich auf rein physikalischem Wege aus der Umgebungsluft gewonnen, so daß ihr späteres Freisetzen keinerlei Verschmutzung oder Beeinträchtigung der Umgebung, sondern lediglich ein Rückführen zu ihrem Ursprung bedeutet.

Weitere Vorteile sind, daß ihr Gebrauch aus dermatologischer wie auch toxikologischer Sicht für den Menschen wie auch für Tiere wohl unbedenklich ist;
schließlich atmet der Mensch täglich ca. 20 Liter
Argon mit der Atemluft ein und aus.

Edelgas besitzt insbesondere für Aerosolerzeugnisse
auf dem Gebiet der Pharmazie den Vorteil, rauschfrei zu wirken. Bekanntlich können Aerosolerzeugnisse, welche Fluor-Chlor-Kohlenwasserstoffe als
Treibmittel enthalten, berauschend wirken. Diese
Wirkung wird von zahlreichen labilen Patienten auch
dazu benutzt, sich vorsätzlich zu berauschen. Nicht
wenige Patienten sind dadurch bereits in eine Abhängigkeit von derartigen Aerosolerzeugnissen geraten, was nicht zuletzt darin zum Ausdruck kommt,
daß bereits entsprechende medizinische Entwöhnungskuren veranstaltet und dafür in Zeitungsinseraten
geworben wird.

Die Bedeutung der Erfindung für Aerosolerzeugnisse
auf dem Gebiet der Pharmazie mag weiter durch die
Tatsache beleuchtet werden, daß heutzutage in einer
normalen Apotheke bereits mehrere Dutzend medizinische Präparate in Aerosolform abgegeben werden, darunter sogenannte Nitrosprays (wie sie bei Verengung der Herzkranzgefäße verordnet werden) Asthmasprays, Rachen- und Mundsprays, Nasensprays, Sprays
zur Behandlung von Ekzemen, Wund- und Wundverbandsprays etc..

Erwähnt sei auch, daß den Edelgasen wohl auch eine
bakterientötende, sterilisierende Wirkung zukommt,
welche in jedem Falle erwünscht ist.

Weiterhin besitzen Edelgase den Vorteil, geruchsneutral zu sein, weshalb sie sich auch besonders für Aerosolerzeugnisse auf dem Gebiet der Kosmetik eignen.

Außerdem führen Edelgase zu einem ausgezeichneten Sprühverhalten bei Aerosolerzeugnissen. Dank ihres guten Lösungsvermögens in Flüssigkeiten lassen sich nämlich in flüssigen Wirkstoffen bzw. den Lösungsmitteln für die Wirkstoffe leicht ausreichende Mengen an Treibmitteln lösen und eine günstige Einstellung der Druckverhältnisse erzielen, d.h. ein mehr oder weniger gleichbleibender Arbeitsdruck und eine gleichmäßige Verteilung (feine Vernebelung).

Schließlich ist der Druckanstieg durch Temperaturerhöhung bei den Edelgasen gering, was noch einen besonderen Sicherheitsfaktor darstellt.

Zweckmäßigerweise ist das Treibmittel ein Gemisch aus den genannten Edelgasen.

Besonders vorteilhaft ist, wenn das Treibmittel ein Gemisch aus Edelgasen mit einem Anteil an Argon von mindestens 80 Gew.%, bezogen auf das Gesamtgewicht des Treibmittels, ist.

Zur Herstellung eines erfindungsgemäßen Aerosolerzeugnisses werden nach einem weiteren Vorschlag der Erfindung Wirkstoff- und Lösungsmittel unter Druck zusammengeführt, über Kopf auf einen Druckbehälter aufgegeben und darin verrieselt. Gleichzeitig wird über Kopf komprimiertes Edelgas, in den Druckbehälter eingeleitet und dem Wirkstoff-Lösungsmittel-
Gemisch zudosiert. Anschließend wird das entstandene Gemisch gesammelt und einer Abfüllanlage zugeleitet. Auf diese Weise läßt sich das
Aerosolerzeugnis in ausreichendem Maße mit Edelgas anreichern.

Vorteilhaft wird das Wirkstoff-Lösungsmittel-Gemisch auf weitere Druckbehälter aufgegeben und wie
zuvor behandelt. Vorzugsweise werden in die Druckbehälter verschiedene Edelgase eingeleitet.

Die erfindungsgemäße Vorrichtung ist gekennzeichnet
durch einen Mischkessel zur Speicherung von Wirkstoff, einen Mischkessel zur Speicherung von Lösungsmittel, wobei die Mischkessel voneinander getrennte
Zuleitungen aufweisen und mit Ableitungen versehen
sind, die zu einer gemeinsamen Förderleitung zusammenlaufen, ferner durch einen Druckbehälter mit einem
Eintrittsstutzen über Kopf, an den die gemeinsame
Förderleitung angeschlossen ist, einem weiteren Eintrittsstutzen über Kopf, an den ein Druckspeicher für
Edelgas angeschlossen ist, sowie einem Abgangsstutzen,
welcher im Abstand vom Boden des Druckbehälters angeordnet ist.

Vorteilhaft sind zusätzliche Druckbehälter vorgesehen.

In weiterer Ausgestaltung des Erfindungsgedankens ist
hinter dem letzten Druckbehälter und/oder vor dem ersten Druckbehälter ein Kerzenfilter vorgesehen.

0011263

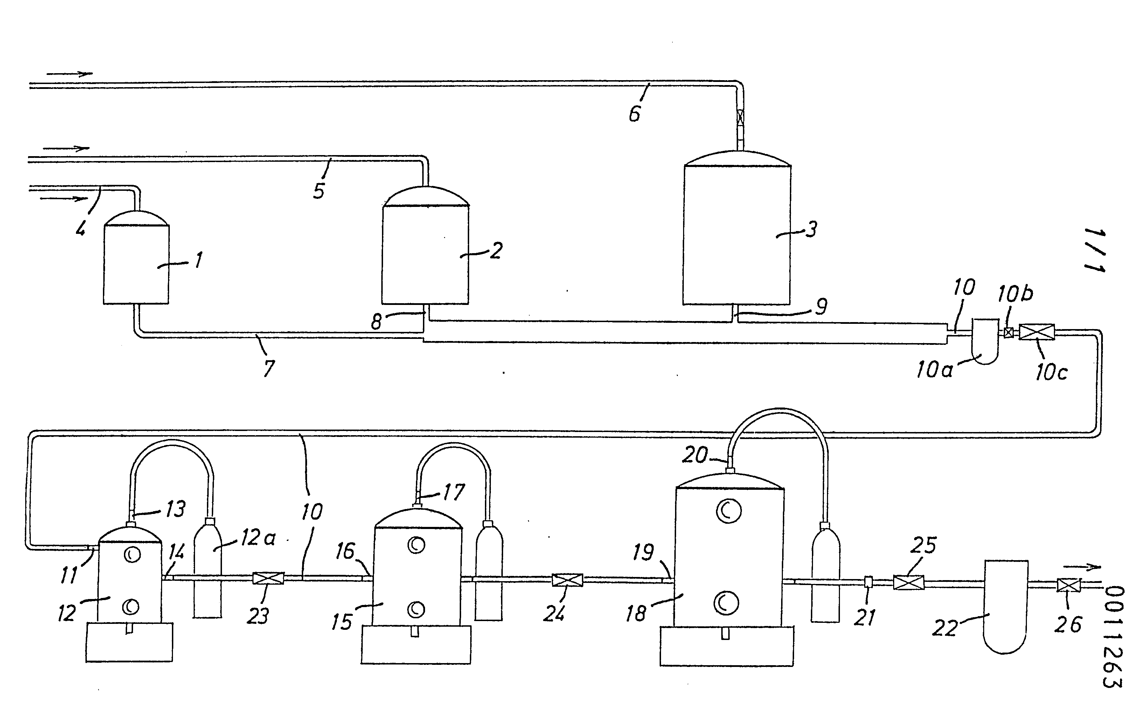

Kurze Beschreibung der Zeichnung

Die Erfindung soll nachstehend in Verbindung mit der Zeichnung weiter erläutert werden. Die einzige Figur zeigt eine erfindungsgemäße Vorrichtung in schematischer Vereinfachung.

Nach der Figur besteht die Vorrichtung aus einem Mischkessel 1 für einen Wirkstoff und einem Mischkessel 2 für einen weiteren Wirkstoff, ferner einem Mischkessel 3 für ein Lösungsmittel. Zu den einzelnen Mischkesseln 1, 2, 3, welche jeweils über ein Rührwerk verfügen, führen getrennte Zuleitungen 4, 5, 6. Von den Mischkesseln 1, 2, 3 gehen Ableitungen 7, 8, 9 ab, die zu einer gemeinsamen Förderleitung 10 zusammenlaufen. Diese weist einen Kerzenfilter 10a, eine Förderpumpe 10b und einen Absperrschieber 10c auf. Die Förderleitung 10 ist weiter an einen Eintrittsstutzen 11 eines Druckbehälters 12 angeschlossen. Der Eintrittsstutzen 11 ist dabei über Kopf des Druckbehälters 12 angeordnet. Ebenfalls über Kopf ist ein weiterer Eintrittsstutzen 13 am Druckbehälter 12 angeordnet, an den ein Druckspeicher (hier eine Druckflasche) 12a für ein Edelgas angeschlossen ist.

Im Abstand zu seinem Boden weist der Druckbehälter 12 einen Abgangsstutzen 14 auf. An diesen schließt sich ein weiterer Abschnitt der Förderleitung 10 an und führt zu einem weiteren Druckbehälter 15, welcher wiederum über einen Eintrittsstutzen 16 für das ankommende Gemisch und einen Eintrittsstutzen 17 für das zuzudosierende Edelgas verfügt. Analog schließt sich daran eine weitere Stufe mit einem Druckbehälter 18 und Eintrittsstutzen 19, 20 an. Die Druckbehälter 12, 15, 18 sind hier als Druckfilterkessel ausgebildet.

Auf den letzten Druckbehälter 18 folgt wiederum ein Abschnitt der Förderleitung 10. In diesen sind ein Überdruckventil 21 sowie ein Kerzenfilter 22 eingebaut. Letzteres besitzt ein durchsichtiges Außengehäuse und ermöglicht gleichzeitig eine visuelle Kontrolle, bevor das gewonnene Gemisch zu einer hier nicht gezeigten Abfüllanlage gelangt. Wie weiter ersichtlich, ist vor und hinter jedem Druckbehälter 12, 15, 18 ein Absperrschieber vorgesehen, nämlich der bereits erwähnte Absperrschieber 10c sowie die weiteren Absperrschieber 23, 24 und 25. Schließlich ist auch hinter dem Kerzenfilter 22 noch ein Absperrschieber angebracht, welcher hier mit 26 bezeichnet ist.

Die erfindungsgemäße Vorrichtung arbeitet beispielsweise wie folgt: Von ihren Mischkesseln 1, 2 bzw. 3 strömen die Wirkstoffe bzw. das Lösungsmittel über ihre dazugehörigen Ableitungen 7, 8 bzw. 9 in die gemeinsame Förderleitung 10. Dort mischen sie sich unter Druck. Das Wirkstoff-Lösungsmittel-Gemisch gelangt sodann zum Druckbehälter 12, wo es über Kopf eintritt und verrieselt. Gleichzeitig wird ebenfalls über Kopf Krypton eingeleitet und dem Gemisch zudosiert.

Das so gewonnene Gemisch gelangt anschließend zu dem Druckbehälter 15, wo in ähnlicher Weise eine Anreicherung mit Helium stattfindet. Daraufhin gelangt jenes Gemisch in den Druckbehälter 18, wo eine noch stärkere Anreicherung mit Argon erfolgt, nämlich mit mindestens 80 Gew.%, bezogen auf das Gesamtgewicht des Treibmittels. Nach Durchlaufen des Kerzenfilters 22 gelangt das erfindungsgemäße Aerosolerzeugnis zur Verpackung in eine Abfüllanlage.

- 10 -

## Ansprüche

1. Aerosolerzeugnis, bestehend aus Wirkstoff, Treibmittel und Lösungsmittel, dadurch gekennzeichnet,
daß das Treibmittel ausschließlich aus Helium, Neon
Argon, Krypton oder Gemischen davon besteht.

2. Aerosolerzeugnis nach Anspruch 1, dadurch gekennzeichnet, daß das Treibmittel ein Gemisch aus den
genannten Edelgasen mit einem Anteil an Argon von
mindestens 80 Gew.%, bezogen auf das Gesamtgewicht
des Treibmittels, ist.

3. Verfahren zur Herstellung eines Aerosolerzeugnisses
nach Anspruch 1, dadurch gekennzeichnet, daß Wirkstoff und Lösungsmittel unter Druck zusammengeführt,
über Kopf auf einen Druckbehälter aufgegeben und
darin verrieselt werden, daß gleichzeitig über Kopf
ein komprimiertes Edelgas in den Druckbehälter eingeleitet und dem Wirkstoff-Lösungsmittel-Gemisch
zudosiert wird und daß anschließend das neu entstandene Gemisch gesammelt und einer Abfüllanlage zugeleitet wird.

4. Verfahren nach Anspruch 3, dadurch gekennzeichnet,
daß das Wirkstoff-Lösungsmittel-Gemisch auf weitere
Druckbehälter aufgegeben und wie zuvor behandelt
wird und daß in die Druckbehälter verschiedene Edelgase eingeleitet werden.

5. Vorrichtung zur Durchführung des Verfahrens nach Anspruch 3, gekennzeichnet durch einen Mischkessel ( 1, 2 ) zur Speicherung von Wirkstoff, einen Mischkessel ( 3 ) zur Speicherung von Lösungsmittel, wobei die Mischkessel ( 1, 2; 3 ) voneinander getrennte Zuleitungen ( 4, 5; 6 ) aufweisen und mit Ableitungen ( 7, 8; 9 ) versehen sind, die zu einer gemeinsamen Förderleitung ( 10 ) zusammenlaufen, ferner gekennzeichnet durch einen Druckbehälter ( 12 ) mit einem Eintrittsstutzen ( 11 ) über Kopf, an den die gemeinsame Förderleitung ( 10 ) angeschlossen ist, einem weiteren Eintrittsstutzen ( 13 ) über Kopf, an den ein Druckspeicher ( 12a ) für Edelgas angeschlossen ist, sowie einem Abgangsstutzen ( 14 ), welcher im Abstand vom Boden des Druckbehälters ( 12 ) angeordnet ist.

6. Vorrichtung nach Anspruch 5, dadurch gekennzeichnet, daß weitere Druckbehälter ( 15, 18 ) vorgesehen sind.

7. Vorrichtung nach Anspruch 5 oder 6, dadurch gekennzeichnet, daß hinter dem letzten Druckbehälter ( 18 ) und/oder vor dem ersten Druckbehälter ( 12 ) ein Kerzenfilter ( 22, 10a ) vorgesehen ist.

1/1

0011263

Europäisches Patentamt

EUROPÄISCHER RECHERCHENBERICHT

0011263
Nummer der Anmeldung

EP 79 10 4434

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | HEALTH PHYSICS London, GB., Vol. 35, Nr. 2, August 1978, Seiten 392-394 Pergamon Press Ltd.: "Fabrication and use of Krypton-85 Aerosol Discharge Devices" <br><br> * Der vollständige Artikel * <br><br> -- | 1 |
| | DE - A - 2 131 668 (INTERCONTINENTAL CHEM.) <br><br> * Seite 9, Zeilen 29-34; Seite 10, Zeilen 1-6; Beispiele * <br><br> -- | 1,3,4 |
| A | DE - B - 1 170 909 (SPECIALTIES DEVEL. CORP.) <br><br> * Spalte 3, Zeilen 11-38 * <br><br> ---- | |

**KLASSIFIKATION DER ANMELDUNG (Int.Cl. 3)**

C 09 K 3/30
A 61 K 9/12

**RECHERCHIERTE SACHGEBIETE (Int. Cl. 3)**

C 09 K 3/30

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 18-12-1979 | BOULON |

EPA form 1503.1  06.78